# EUROPEAN PATENT APPLICATION

(11) **EP 4 173 656 A1**
(43) Date of publication of application: **03.05.2023**
(21) Application number: 22187583.4
(22) Date of filing: 28.07.2022
(51) Int. Cl.: A61M 5/20, A61M 5/32

(54) **NEEDLE GUARD, DRUG DELIVERY DEVICE AND METHOD FOR MANUFACTURING**

(30) Priority: 27.10.2021 EP 21205085; 23.12.2021 EP 21217555; 14.04.2022 EP 22168393
(71) Applicant: medmix Switzerland AG, 9469 Haag (Rheintal) (CH)
(72) Inventor: Marsh, William Geoffrey Arthur, Stratford-upon-Avon (GB); Morris, Anthony Paul, Balsall Common (GB); Jones, Matthew Meredith, Warwick (GB); James, Aled Meredydd, Solihull (GB)
(74) Representative: Manitz Finsterwald Patent- und Rechtsanwaltspartnerschaft mbB

(57) **Abstract**

The present invention refers to a needle guard 9A for a drug delivery device 1, wherein the needle guard 9A comprises a proximal sleeve part 17 for shielding a needle 5B, and a distal arm part 21 for activating a dispense operation of the drug delivery device. Said proximal sleeve part 17 and said distal arm part 21 are separate components connected to each other, in particular in a non-releasable manner. Besides, the present invention refers to a drug delivery device 1 provided with such a needle guard 9A and a method for manufacturing such a needle guard 9A and/or drug delivery device 1.

## Description

The present invention relates to a needle guard for a drug delivery device, a drug delivery device with such a needle guard and a method for manufacturing such a needle guard or drug delivery device.

Drug delivery devices are known in various configurations and for various areas of application. Thus, the components of such drug delivery devices are under permanent development to meet the various demands defined by the intended areas of application. Besides the basic functional requirements defined by the intended area of application, in particular, the simplicity and cost efficiency of manufacturing and/or the user friendliness are important aspects to be considered during the development of the components of such drug delivery devices. One specific component of such drug delivery devices is the needle guard, in particular if said needle guard not only serves for shielding the needle of the drug delivery device but also serves as an activation component for activating the delivery operation upon movement with respect to the housing of the drug delivery device.

In view of this, it is an object of the present invention to provide an improved configuration for such a needle guard, in particular in view of manufacturing simplicity and efficiency and/or user friendliness. This object is solved by the subject matter of the independent claim 1. The further independent claims refer to a corresponding drug delivery device and corresponding methods for manufacturing. Preferable modifications and/or improvements are given in the dependent claims.

According to the present invention, a needle guard for a drug delivery device comprises a proximal sleeve part for shielding a needle and a distal arm part for activating a dispense operation of the drug delivery device. Said proximal sleeve part and said distal arm part are separate components connected to each other, in particular in a non-releasable manner.

Such a two-part configuration of the needle guard simplifies the manufacturing of the needle guard, while enhancing the flexibility in designing the configuration of the two components of the needle guard substantially. Over the known single-part implementations, the two-part form configuration allows an easy manufacturing of a needle guard with different properties for the two components thereof, i.e. of the sleeve part and of the arm part. Some examples for this flexibility are given in the following. Moreover, said two-part configuration allows the implementation of a modular system for the needle guard reducing the manufacturing costs. Finally, such a two-part configuration can simplify the assembling process of the drug delivery device substantially.

Preferably, the proximal sleeve part and/or the distal arm part are molded components.

Molded components of low complexity are quite cheap in general. More complex forms for such components result in substantially increased efforts and costs for production thereof. The two-part configuration allows to separate the molding processes for the two components of the needle guard and, thus, increases the flexibility in the design for the two components as well as for the overall needle guard, while keeping low the efforts and costs for production.

Preferably, the proximal sleeve part and the distal arm part are formed of different materials.

Thus, specific requirements to be met by the needle guard can be met by freely adjusting the material for the two components thereof.

The proximal sleeve part can be formed of a softer material than the distal arm part.

As the sleeve part is provided to contact the injection site, in general the skin of a user, such a softer material improves the wellbeing of a user of the drug delivery device, while the harder material for the arm part ensures the internal functionality of the needle guard. Alternatively, the exposed part (i.e. the sleeve part) may be formed in a very impact resistant material to improve robustness in the event of free-fall impact.

Preferably, the distal arm part is formed of a rigid but elastically deformable material.

Such a configuration results in a reliable and functional configuration for the needle guard.

Preferably, the proximal sleeve part and the distal arm part are provided in different colors.

Such a configuration allows to combine various counterpart aspects related to the color of the needle guard.

Preferably, the proximal sleeve part is provided in a bright color, like yellow, red, purple, blue or green.

This configuration allows a user to easily determine the relative position of the sleeve part of the needle guard with respect to the housing, in particular if said color differs substantially from the color of the housing of the drug delivery device. Moreover, the user can clearly differentiate with respect to the cap of the device which covers the proximal sleeve of the needle guard before use.

Preferably, the distal arm part is provided in a neutral color, like black, white or grey.

Such a configuration allows to prevent color-related irritations when looking through a window of the drug delivery device and determining the color or charging level of a syringe. Moreover, such a configuration enables a visual check of the clarity of the fluid prior to injection, which is required with some medicaments.

Preferably, the proximal sleeve part comprises at least one, in particular two, radially extending cap detents for engaging and holding a cap to be coupled to the drug delivery device.

Thus, the cap can be coupled directly to the needle guard and can be used to hold or fix the needle guard in its proximal position until the cap is removed from the needle guard.

Preferably, the distal arm part comprises at least one, in particular two, trigger ramps configured to release in the distal activation position of the needle guard a dispense mechanism of the drug delivery device for activating the dispense operation.

Such trigger ramps are easy to form and result in a very reliable activation of the dispense operation upon distal movement of the needle guard.

Further preferably, the at least one trigger ramp is provided at a distal end section of the distal arm part.

Thus, the whole length of the arm part is used, which allows a quite compact but still reliable overall configuration.

Preferably, the distal arm part comprises at least one, in particular two, lockout arms configured to engage with the housing in a post-delivery state of the drug delivery device for locking the needle guard in a proximal shielding position.

Thus, a de-shielding distal movement of the needle guard uncovering the needle of the drug delivery device after the completed dispense operation is prevented reliably.

Preferably, the proximal sleeve part and the distal arm part are connected to each other via a snap-fit connection.

Such a snap-fit connection is quite simple but reliable for connecting the sleeve part and the arm part to each other to form the needle guard.

A further aspect of the present invention refers to a drug delivery device. According to the present invention, such a drug delivery device comprises a housing, a syringe, a dispense mechanism as well as a shielding and activation mechanism. The housing has a proximal end and a distal end. The syringe comprises a container filled with a drug to be delivered and a needle through which the drug is to be delivered. The dispense mechanism is preloaded but locked and provided for dispensing the drug from the syringe. The shielding and activation mechanism is configured to activate the dispense operation of the dispense mechanism and to shield the needle before and after the dispense operation of the dispense mechanism. The shielding and activation mechanism comprises one of the above described needle guards. Said needle guard is configured to be movable between a proximal shielding position and a distal activation position with respect to the housing.

Such a configuration allows to make use of the above described advantages of the provided needle guard in a drug delivery device.

Preferably, the drug delivery device is an autoinjector, in particular a single-use fixed dose autoinjector.

The present needle guard was developed in particular in view of the requirements of such types of drug delivery devices.

A last aspect of the present invention refers to a method for manufacturing the above described needle guards or the above described drug delivery devices. According to the present invention, such a method comprises the following steps: forming the proximal sleeve part and the distal arm part of the needle guard in separate processes, and connecting the proximal sleeve part and the distal arm part to each other, in particular in a non-releasable manner, to form the needle guard before or during the at least partial insertion thereof into the housing.

Such a method is quite simple while allowing to make use of the advantages of the provided needle guard during the manufacturing process.

An exemplary embodiment and functions of the present invention are described in the following referring to the accompanying drawings, wherein
- FIG. 1: shows a sectional view of an exemplary drug delivery device configured to be provided with the needle guard of the present invention in a pre-delivery state;
- FIGS. 2A and 2B: show sectional views of the drug delivery device of FIG. 1 in two states during a dispense operation;
- FIG. 3: shows an exploded view of the drug delivery device of FIG. 1;
- FIGS. 4a to 4D: show the activation functionality of the drug delivery device of FIG. 1;
- FIG. 5: shows an outer body of the housing of the drug delivery device of FIG. 1 in more detail;
- FIG. 6: shows a perspective view of a needle guard in accordance with the present invention configured to be used with the drug delivery device of FIG. 1;
- FIG. 7: shows a perspective view of the distal arm part of the needle guard of FIG. 6; and
- FIG. 8: shows a proximally directed view on the proximal sleeve part of the needle guard of FIG. 6.

In the present document, the term "distal" refers to the part/end of the device, or the parts/ends of the components or members thereof, which during the use of the device, is located the furthest away from a delivery/injection site of a patient. Correspondingly, the term "proximal" refers to the part/end of the device, or the parts/ends of the members thereof, which during the use of the device is located closest to the delivery/injection site of the patient.

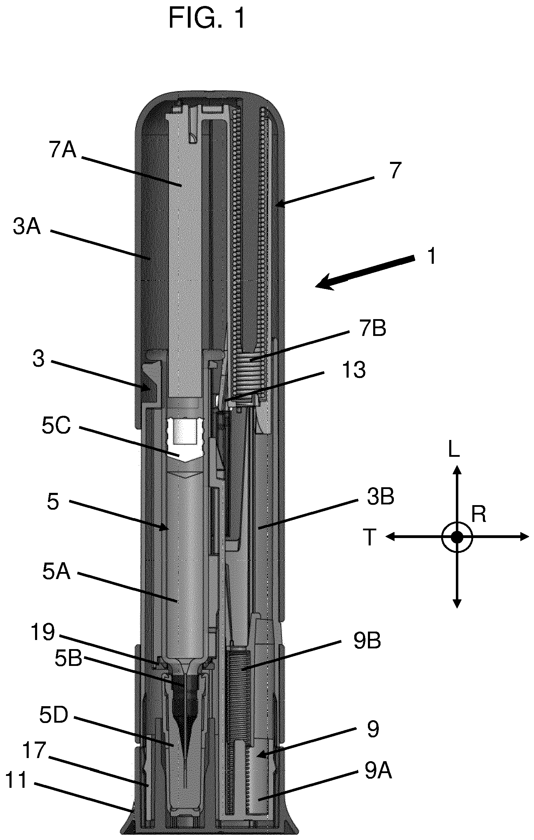

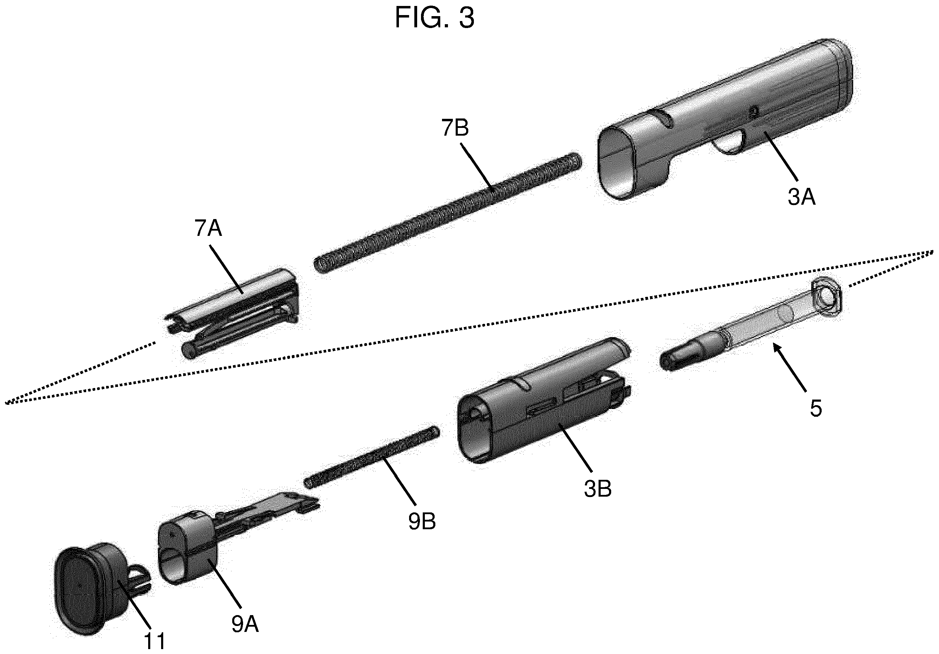

The present disclosure of the needle guard is applicable with various drug delivery devices. One exemplary drug delivery device is described in the following with respect to FIGS. 1 to 5.

A here illustrated drug delivery device 1 comprises a housing 3 having a proximal end (at the bottom of FIG. 1) as well as a distal end (on the top of FIG. 1). The housing 3 is formed of an outer body 3A and an inner body 3B connected to each other in a non-movable manner in particular via a snap-fit connection. A syringe 5 comprising a container 5A filled with a drug to be delivered, a needle 5B, a movable stopper 5C and a rigid needle shield 5D, is positioned in a substantially non-movable manner within said housing 3. Moreover, the drug delivery device 1 comprises a dispense mechanism 7 formed of a U-shaped drive chassis 7A positioned movable along the housing 3 within the housing 3, and a drive spring 7B for moving the drive chassis 7A in the proximal direction to dispense the drug from the syringe 5. Furthermore, the drug delivery device 1 comprises a shielding and activation mechanism 9 comprising of a needle guard 9A and a needle guard spring 9B. Finally, a cap 11 is provided on the proximal end of the drug delivery device 1. Fig. 1 shows the drug delivery device 1 in its pre-delivery state as supplied to a user of the drug delivery device 1.

In the following, the internal structural as well as functional configuration of the illustrated drug delivery device 1 is described roughly.

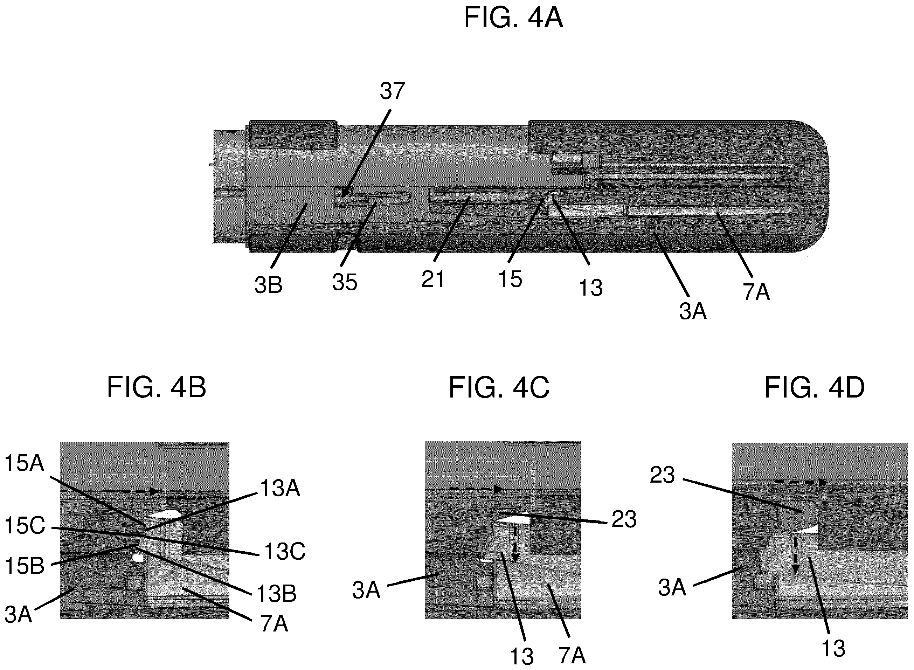

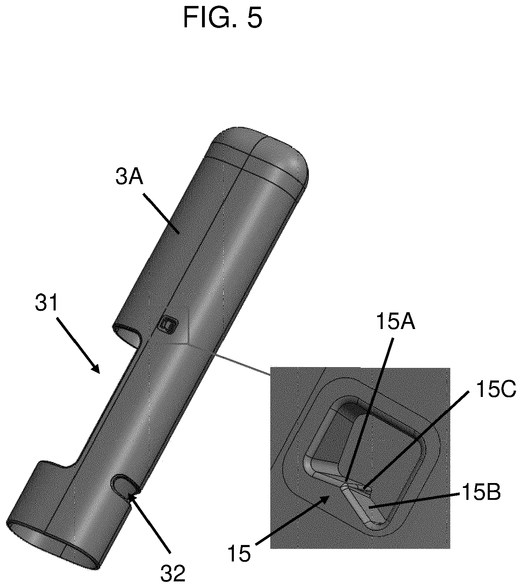

In the supplied pre-delivery state illustrated in FIG. 1, the drive chassis 7A is locked against proximal movement with respect to the housing 3 by at least one trigger arm 13 engaging a corresponding trigger stop 15 provided in the outer body 3B of the housing 3. Said engaging configuration can be seen best in FIG. 4B, while an enlarged view of said trigger stop 15 is illustrated in FIG. 5. The trigger arm 13 comprises a first contact surface 13A and a second contact surface 13B separated from each other by a step 13C. The trigger stop 15 comprises a corresponding first contact surface 15A and a corresponding second contact surface 15B separated from each other by a corresponding step 15C. As can be seen both in FIGS. 4A to 4D and FIG. 5, the first surfaces 13A and 15A and the second surfaces 13B and 15B have different orientations or slopes, while the second surfaces 13B and 15B are steeper than the first surfaces 13A and 15A. In particular, as can be seen in FIG. 5 said contact surfaces 13A, 13B, 15A and 15B are tilted against each other in more than one rotational direction and form arrow-shaped contact areas. In the initial pre-delivery state, the first surfaces 13A and 15A contact each other and the second surfaces 13B and 15b contact each other. In particular, the contact surfaces 15A and 15B of the trigger stop 15 form a distally pointing stepped arrow-shaped protrusion and the contact surfaces 13A and 13B of the trigger arm 13 form a distally pointing stepped arrow-shaped cut-out formed inversely to the distally pointing stepped arrow-shaped protrusions formed by the contact surfaces 15A and 15B of the trigger stop 15.

A longitudinal direction L, a radial direction R and a transversal direction T of the drug delivery device 1 are indicated in FIG. 1. The longitudinal direction L extends along a length of a drug delivery device 1 in parallel to the drive spring 7B. The transverse direction T extends perpendicular thereto along a width of the drug delivery device 1 and the radial direction R extends perpendicular thereto out of the page.

Alternatively, a configuration with a reversed orientation can be implemented. In particular, the contact surfaces 15A and 15B can form a proximally pointing arrow-shaped cut-out and contact surfaces 13A and 13B can form a proximally pointing arrow-shaped protrusion.

In the following, the use of such a drug delivery device 1 is described.

For preparing the delivery of the drug, the cap 11, particularly locking via detents a distal movement of the needle guard 9A with respect to the housing 3, is pulled in the proximal direction (i.e. to the bottom of FIG. 1) from a proximal sleeve part 17 of the needle guard 9A. By this, the rigid needle shield 5D, which is engaged by clipping arms 19 of the cap 11 is removed from the needle 5B.

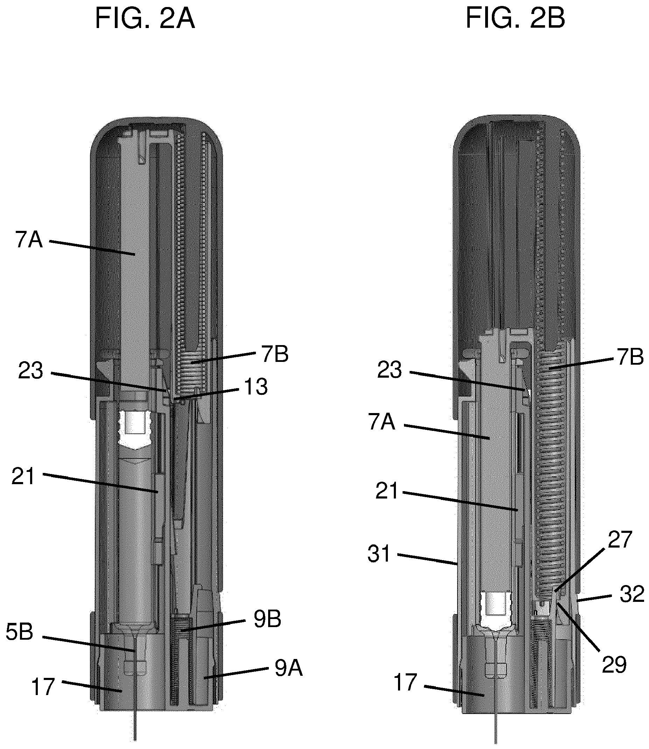

Then, the drug delivery device 1 is pushed with the proximal sleeve part 17 of the needle guard 9A against the injection site resulting in a distal movement of the needle guard 9A into the housing 3 against the force of the needle guard spring 9B. This movement results in a revealing of the needle 5B and an insertion thereof into the injection site. At the same time, a distal arm part 21 of the needle guard 9A moves distally along the housing 3 resulting in trigger ramps 23 provided on a distal end section of the distal arm part 21, pushing the corresponding trigger arms 13 in a transversal direction T for disengaging the respective trigger arms 13 from the corresponding trigger stops 15. This pushing process is illustrated in FIGS. 4B to 4D (see in particular the dashed arrows) and the finally disengaged state is illustrated in FIG. 2A.

During said disengagement movement, the contact surfaces 13A and 13B of the trigger arms 13 are pushed along the corresponding contact surfaces 15A and 15B of the trigger stops 15 in a transversal direction T until the end of the first contact surfaces 13A of the trigger arms 13 pass the steps 15C of the corresponding trigger stops 15. When the contact surfaces 13A of the trigger arms 13 have passed the steps 15C of the corresponding trigger stops 15, the force from the drive spring 7B causes the trigger arms 13 to deflect further as they follow the angled contact surfaces 15B of the trigger stops 15. As soon as the contact surfaces 13A have passed the end of the angled contact surfaces 15B the drive chassis 7A is fully released and drive spring 7B pushes the drive chassis 7A in the proximal direction for the proximal dispense movement of the drive chassis 7A.

During this proximal movement of the drive chassis 7A, a plunger rod section of the drive chassis 7A contacts and pushes the stopper 5C in the proximal direction for dispensing the drug from the container 5A through the needle 5B.

Near the end of this dispense movement of the drive chassis 7A, an end of dose click arm 27 provided on the inner body 3B of the housing 3 snaps over an end of dose click ramp 29 of the drive chassis 7A generating an acoustic end of dose click for the user. For monitoring said dispense movement, the housing 3 of the drug delivery device 1 comprises a first state indication window 31 and a second state indication window 33. The first indication window 31 shows the container 5A of the syringe 5 and thus also the piston rod section of the drive chassis 7A moving through the container 5A during the dispense operation. The second window 32 is positioned in a proximal section of the housing 3 and configured such that any section of the drive chassis 7A is only visible therethrough at or near the end of the dispense operation (see FIG. 2B).

After the full dispense operation is completed, the drug delivery device 1 is removed from the injection site resulting in the needle guard spring 9B moving the needle guard 9A back into a proximal position with respect to the housing 3 shielding the needle 5B. As it approaches its proximal end position, the trigger arms 13 of the drive chassis 7A force corresponding lockout arms 35 provided on the needle guard 9A to engage with a shoulder of a lockout slot 37 (see FIG. 4A) provided in the inner body 3a of the housing 3. Thus, the needle guard 9A is locked against a repeated movement in the distal direction. In this state, the cap 11 with the rigid needle shield 5D can be put on the needle guard 9A again for further security, if desired.

As can be taken from the above functional description, the illustrated and described drug delivery device 1 is a single-use fixed dose autoinjector. However, the specific configuration for the needle guard 9A described in the following can be also used in drug delivery devices 1 of other types.

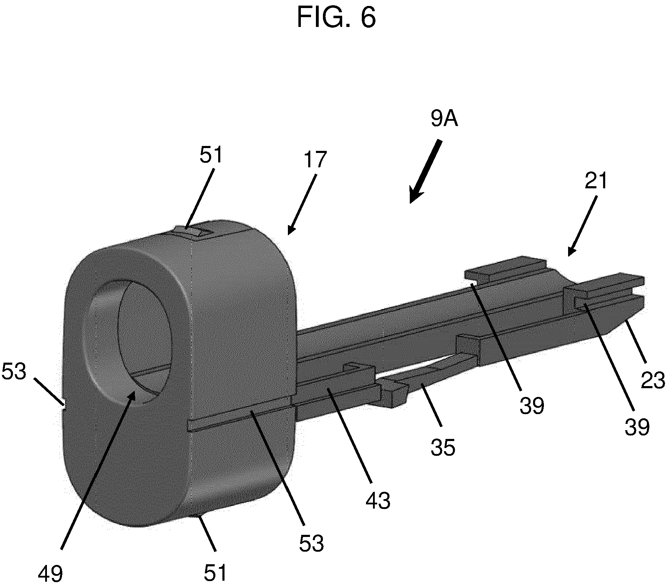

In the following, the structural and functional configuration of the needle guard 9A in accordance with the present invention will be described in more detail with respect to FIGS. 6 to 8. Such a two-part needle guard 9A can be used instead of the single-part needle guard 9A illustrated in the above described figures without any modifications to the other components of the drug delivery device 1.

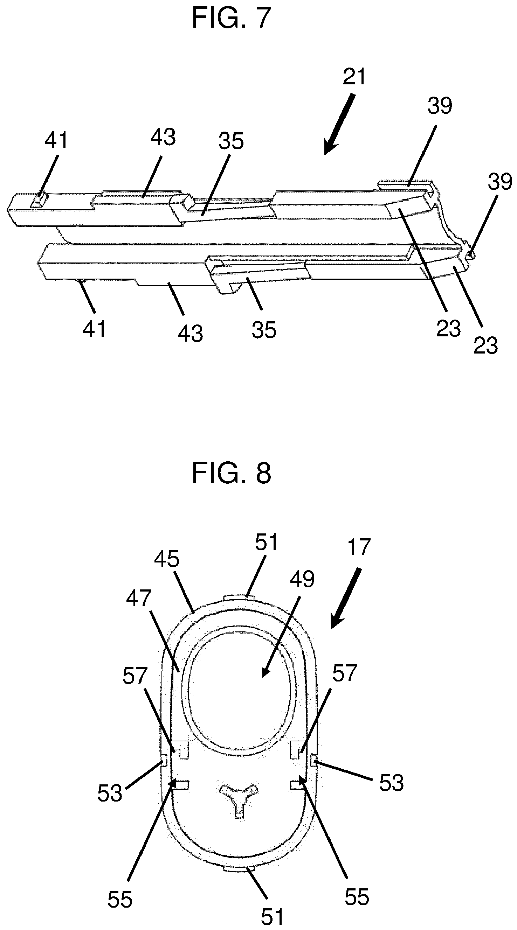

The needle guard 9A is formed of a proximal sleeve part 17 (see FIG. 8) and a distal arm part 21 (see FIG. 7) connected to each other, in particular non-releasably, during assembly.

As can be seen best in FIG. 7, the arm part 21 comprises a distal end section (on the right) on which two trigger ramps 23 for releasing the trigger arms 13 of the drive chassis 7A are provided. Moreover, said end section of the distal arm part 21 comprises two axially extending guide slots 39 configured to engage guide rails of the housing 3. In a central section of the arm part 21, the above noted lockout arms 35 are provided. Each of the lockout arms 35 has a L-shaped form with a radially (i.e. along a radial direction R) extending protrusion configured to engage the lockout slot 37 of the inner body 3B of the housing 3 (see FIG. 4A). At the proximal end section (on the left) of the arm part 21, two snap-in protrusions 41 configured to snap-in with snap-in recesses (not illustrated) of the sleeve part 17. Between the lockout arms 35 and the snap-in protrusions 41, the arm part 21 is provided with radially extending spline features 43 for engagement with the inner body 3B.

As can be seen in FIG. 8, the sleeve part 17 has a cap like overall shape with a circumferential side wall 45 and a flat end wall 47 connected to said side wall 45. The end wall 47 is provided with a needle opening 49. The outer surface of the side wall 45 is provided with two cap detents 51 configured to engage corresponding detents (not illustrated) provided within the cap 11, and two axially extending guide recesses 53 for engagement with corresponding guide protrusions (not illustrated) of the housing 3, in particular of the inner body 3B of the housing 3. The inner surface of the side wall 45 comprises two receiving sections 55 for receiving and holding the distal arm part 21 of the needle guard 9A. Said receiving sections 55 are provided with receiving recesses 57 for the distal arm part 21 and with snap-in recesses (not illustrated) for forming a snap-fit connection with the snap-in protrusions 41 of the distal arm part 21 to connect the proximal sleeve part 17 and the distal arm part 21 to each other, in particular in a non-releasable manner.

Preferably, at least one, in particular both of the proximal sleeve part 17 and the distal arm part 21 are molded components. If desired, said two components 17 and 21 can be formed of different materials, wherein in particular, the material of the proximal sleeve part 17 is quite soft for improved feeling on the skin of a user, while the material of the distal arm part 21 is rigid but elastically deformable for ensuring the functionality of this component.

Furthermore, said two components 17 and 21 of the needle guard 9A can be provided in different colors, while in particular, the proximal sleeve part 17 is provided in a bright color, like yellow, red, purple, blue or green, and the distal arm part 11 is provided in a neutral color, like black, white or grey.

A method for manufacturing such a needle guard 9A comprises the formation of the sleeve part 17 and of the arm part 21 in separate processes and an in particular non-releasable connection of these two components 17 and 21.

A further aspect developed together with the present invention is summarized with reference to the following embodiments.
1. A drug delivery device (1) comprising:
   a housing (3) having a proximal end and a distal end;
   a syringe (5) comprising a container (5A) filled with a drug to be delivered and a needle (5B) through which the drug is to be delivered;
   a dispense mechanism (7) for dispensing the drug from the drug delivery device (1); and
   a shielding and activation mechanism (9) configured to activate a dispense operation of the dispense mechanism (7) and to shield the needle (5B) before and after the dispense operation of the dispense mechanism (7);
   wherein the dispense mechanism (7) comprises a drive chassis (7A) and a drive spring (7B);
   wherein the drive chassis (7A) is movable between a distal pre-delivery position and a proximal post-delivery position through the housing (3); wherein the drive spring (7B) is positioned between the drive chassis (7A) and a distal wall of the housing (3) to bias the drive chassis (7A) from its distal pre-delivery position to its proximal post-delivery position; wherein the drive chassis (7A) comprises at least one trigger arm (13) engaging a trigger stop (15) of the housing (3) in the pre-delivery position of the drive chassis (7A) for locking the drive chassis (7A) against the force of the drive spring (7B);
   wherein each trigger arm (13) and corresponding trigger stop (15) comprises an axially oriented contact area abutting each other in the pre-delivery state;
   **characterized in that**
   each of the contact areas is formed of a first contact surface (13A, 15A) and a second contact surface (13B, 15B) separated from each other by a step (13C, 15C).
2. The drug delivery device (1) of embodiment 1,
   wherein the first contact surfaces (13A, 15A) and the second contact surfaces (13B, 15B) have different slopes.
3. The drug delivery device (1) of embodiment 2,
   wherein the slope of the second contact surfaces (13B, 15B) is steeper than the one of the first surfaces (13A, 15A).
4. The drug delivery device (1) of any one of the preceding embodiments, wherein the contact surfaces (15A, 15B) of the trigger stops (15) form a distally pointing stepped arrow-shaped protrusion and the contact surfaces (13A, 13B) of the trigger arms (13) form distally pointing stepped arrow-shaped cut-outs formed inversely to the distally pointing stepped arrow-shaped protrusions formed by the contact surfaces (15A, 15B) of the trigger stops (15).
5. The drug delivery device (1) of any one of the preceding embodiments, wherein the shielding and activation mechanism (9) comprises a needle guard (9A) movable from a proximal shielding position to a distal activation position with respect to the housing (3),
   wherein upon said movement, the needle guard (9A) causes disengagement of the trigger arms (13) from the corresponding trigger stops (15).
6. The drug delivery device (1) of embodiment 5,
   wherein the needle guard (9A) comprises one trigger ramp (23) for each trigger arm (13) of the drive chassis (7A) configured to move the corresponding trigger arm (13) in a transversal direction (T) for disengaging the respective trigger arm (13) from the corresponding trigger stop (15).
7. The drug delivery device (1) of embodiment 5 or 6,
   wherein the drug delivery device (1) is configured such that the contact surfaces (13A, 13B) of the trigger arms (13) are pushed along the corresponding contact surfaces (15A, 15B) of the trigger stops (15) in a transversal direction (T) during the disengaging movement until the end of the first contact surfaces (13A) of the trigger arms (13) pass the steps (15C) of the corresponding trigger stops (15),
   as soon as the first contact surfaces (13A) of the trigger arms (13) have passed the steps (15C) of the corresponding trigger stops (15), the force from the drive spring (7B) causes the trigger arms (13) to deflect further as they follow the angled contact surfaces (15B) of the trigger stops (15) and as soon as the contact surfaces (13A) have passed the end of the angled contact surfaces (15B) the drive chassis (7A) is fully released and the proximal dispense movement of the drive chassis (7A) is caused by the drive spring (7B).
8. The drug delivery device (1) of any one of the preceding embodiments, wherein the contact areas (13A, 13C) of the trigger arms (13) are provided on a proximal section of the drive chassis (7A).
9. The drug delivery device (1) of any one of the preceding embodiments, wherein the trigger arms (13) are configured to interact with the shielding and activation mechanism (9) not just for releasing the drive chassis (7A) but also for locking the shield and activation mechanism (9) in a post-delivery state.
10. The drug delivery device (11) of any one of the preceding embodiments, wherein the drive chassis (7A) comprises a spring chassis section for enclosing the drive spring (7B), the trigger arms (13) and a plunger rod section, all of these components being formed as integral single part component.
11. The drug delivery device (1) of any one of the preceding embodiments, wherein the drive chassis (7A) is U-shaped seen along a radial direction (R) of the drug delivery device (1).
12. The drug delivery device (1) of any one of the proceeding embodiments, wherein the housing (3) comprises at least one state indication window (31, 32) provided in a proximal section of the housing (3),
   wherein the drug delivery device (1) is configured such that in the pre-delivery state, no component of the drive chassis (7A) is visible through the at least one state indication window (31, 32), while in the post-delivery state, a section of the drive chassis (7A) is visible through said at least one state indication window (31, 32).
13. The drug delivery device (1) of embodiment 12,
   wherein the housing (3) comprises two of said state indication windows (31, 32),
   wherein in the pre-delivery state of the drug delivery device (1), through a first state indication window (31) thereof, the container (5A) of the syringe (5) and the drug contained therein is visible, while through none of both state indication windows (31, 32), any section of the drive chassis (7A) is visible, and
   in the post-delivery state of the drug delivery device (1), through the first state indication window (31), a piston rod section of the drive chassis (7A) inserted into the container (5A) of the syringe (5) is visible, and through a second state indication window (32) another section of the drive chassis (7A) is visible.

### Reference numeral list

- 1: drug delivery device
- 3: housing
- 3A: outer body
- 3B: inner body
- 5: syringe
- 5A: container
- 5B: needle
- 5C: stopper
- 5D: needle shield
- 7: dispense mechanism
- 7A: drive chassis
- 7B: drive spring
- 9: shielding and activation mechanism
- 9A: needle guard
- 9B: needle guard spring
- 11: cap
- 13: trigger arm
- 13A: first contact surface
- 13B: second contact surface
- 13C: step
- 15: trigger stop
- 15A: first contact surface
- 15B: second contact surface
- 15C: step
- 17: proximal sleeve part
- 19: clipping arms
- 21: distal arm part
- 23: trigger ramp
- 27: end of dose click arm
- 29: end of dose click ramp
- 31: first state indication window
- 32: second state indication window
- 35: lockout arm
- 37: lockout slot
- 39: guide slots
- 41: snap-in protrusion
- 43: spline feature
- 45: side wall
- 47: end wall
- 49: needle opening
- 51: cap detent
- 53: guide recess
- 55: receiving section
- 57: receiving recess

## Claims

1. A needle guard (9A) for a drug delivery device (1), the needle guard (9A) comprising:
a proximal sleeve part (17) for shielding a needle (5B); and
a distal arm part (21) for activating a dispense operation of the drug delivery device (1),
**characterized in that**
said proximal sleeve part (17) and said distal arm part (21) are separate components connected to each other, in particular in a non-releasable manner.

2. The needle guard (9A) according to claim 1,
wherein the proximal sleeve part (17) and/or the distal arm part (21) are molded components.

3. The needle guard (9A) according to claim 1 or 2,
wherein the proximal sleeve part (17) and the distal arm part (21) are formed of different materials.

4. The needle guard (9A) of claim 3,
wherein the proximal sleeve part (17) is formed of a softer material than the distal arm part (21).

5. The needle guard (9A) of any one of the preceding claims,
wherein the distal arm part (21) is formed of a rigid but elastically deformable material.

6. The needle guard (9A) of any one of the preceding claims,
wherein the proximal sleeve part (17) and the distal arm part (21) are provided in different colors.

7. The needle guard (9A) of any one of the preceding claims,
wherein the proximal sleeve part (17) is provided in a bright color, like yellow, red, purple, blue or green.

8. The needle guard (9A) of any one of the preceding claims,
wherein the distal arm part (21) is provided in a neutral color, like black, white or grey.

9. The needle guard (9A) of any one of the preceding claims,
wherein the proximal sleeve part (17) comprises at least one, in particular two, transversally extending cap detents (51) for engaging and holding a cap (11) to be coupled to the drug delivery device (1).

10. The needle guard (9A) of any one of the preceding claims,
wherein the distal arm part (21) comprises at least one, in particular two, trigger ramps (23) configured to release in the distal activation position of the needle guard (9A) a dispense mechanism (7) of the drug delivery device (1) for activating the dispense operation.

11. The needle guard (9A) of claim 10,
wherein the at least one trigger ramp (23) is provided at a distal end section of the distal arm part (21).

12. The needle guard (9A) of any one of the preceding claims,
wherein the distal arm part (21) comprises at least one lockout arm (35) configured to engage with the housing (3) in a post-delivery state of the drug delivery device (1) for locking the needle guard (9A) in a proximal shielding position.

13. The needle guard (9A) of any one of the preceding claims,
wherein the proximal sleeve part (17) and the distal arm part (21) are connected to each other via a snap-fit connection.

14. A drug delivery device (1) comprising:
a housing (3) having a proximal end and a distal end;
a syringe (5) comprising a container (5A) filled with a drug to be delivered and a needle (5B) through which the drug is to be delivered;
a preloaded but locked dispense mechanism (7) for dispensing the drug from the syringe (5); and
a shielding and activation mechanism (9) configured to activate the dispense operation of the dispense mechanism (7) and to shield the needle (5B) before and after the dispense operation of the dispense mechanism (7);
**characterized in that**
the shielding and activation mechanism (9) comprises the needle guard (9A) according to any one of the preceding claims,
wherein the needle guard (9A) is configured to be movable between a proximal shielding position and a distal activation position with respect to the housing (3).

15. The drug delivery device (1) according to claim 14,
wherein the drug delivery device (1) is an autoinjector, in particular a single-use fixed dose autoinjector.

16. A method for manufacturing the needle guard (9A) of any one of the preceding claims 1 to 13 or the drug delivery device (1) of any one of the preceding claims 14 and 15, the method comprising the following steps:
forming the proximal sleeve part (17) and the distal arm part (21) of the needle guard (9A) in separate processes;
connecting the proximal sleeve part (17) and the distal arm part (21) to each other, in particular in a non-releasable manner, to form the needle guard (9A) before or during the at least partial insertion thereof into the housing (3).
